# EUROPÄISCHE PATENTANMELDUNG

(11) **EP 2 238 940 A1**
(43) Veröffentlichungstag der Anmeldung: **13.10.2010**
(21) Anmeldenummer: 10003645.8
(22) Anmeldetag: 01.04.2010
(51) Int. Cl.: A61B 19/00, A61B 17/225, A61H 23/00

(54) **Verfahren zur Vorbereitung eines Geräts zur Behandlung des menschlichen oder tierischen Körpers mit mechanishen Druckwellen**

(30) Priorität: 03.04.2009 DE 102009016102
(71) Anmelder: STORZ MEDICAL AG, 8274 Tägerwilen (CH)
(72) Erfinder: Novak, Pavel, Dr., 8234 Stetten, SH (CH); Schulz, Manfred, 8274 Tägerwilen/T (CH); Froese, Klaus, 78465 Konstanz (DE)
(74) Vertreter: Szynka, Dirk

(57) **Zusammenfassung**

Die Erfindung betrifft ein Verfahren zur regelmäßigen Vorbereitung eines Geräts zur Behandlung des menschlichen oder tierischen Körpers durch mechanische Druckwellen auf eine solche Behandlung, bei dem ein zur Druckwellenerzeugung verwendeter Prallkörper (9) vor jeder Behandlung eines weiteren Patienten gegen einen unbenutzten Prallkörper (9) ausgetauscht wird. Der Prallkörper kann als Einwegpartikel ausgestaltet sein.

## Beschreibung

Diese Erfindung bezieht sich auf die Vorbereitung eines zur Behandlung des menschlichen oder tierischen Körpers durch mechanische Druckwellen ausgelegten Geräts und auf ein entsprechendes Gerät selbst sowie dessen Verwendung.

Geräte zur Behandlung mit mechanischen Druckwellen sind an sich bereits bekannt, insbesondere aus dem Bereich der Lithotripsie. Dort werden mit fokussierten mechanischen Druckwellen Körperkonkremente, insbesondere Steine im Körpergewebe, zertrümmert. Neben der Erzeugung durch elektrische Entladungen in Wasser sind auch Geräte entwickelt worden, die mechanische Druckwellen durch das Aufeinanderprallen eines beschleunigten Schlagteils und eines Prallkörpers erzeugen und mit Hilfe des Prallkörpers in Körpergewebe einkoppeln. Solche Geräte sind sowohl in der Lithotripsie mit einem direkten Kontakt zwischen dem Prallkörper bzw. einer mit dem Prallkörper verbundenen Sonde und dem Stein als auch bei anderen Behandlungen von biologischen Körpersubstanzen eingesetzt worden. Insbesondere sind hier die Behandlung von Muskelerkrankungen und von Erkrankungen im Übergangsbereich zwischen Muskeln und Knochen zu nennen.

Ein Beispiel für ein Gerät, das zu dem zuletzt genannten Typ zu rechnen ist, ist die in der EP 0 991 447 dargestellte Vorrichtung. Bei dieser sollen unfokussierte Druckwellen in das Körpergewebe eingekoppelt werden, indem das Gerät von außen auf die Körperoberfläche aufgesetzt wird.

Bei solchen Geräten kann in vielen Fällen die Heftigkeit der eingekoppelten Druckwelle durch Einstellung eines Druckwerts einer pneumatischen Versorgungseinrichtung verändert werden. Je höher der anstehende Pneumatikdruck, umso heftiger wird das Schlagteil beschleunigt und umso größer ist der impuls- und Energieübertrag auf den Prallkörper.

Überdies erlauben viele Geräte die Einstellbarkeit der Wiederholfrequenzen pneumatischer Pulse und damit der Wiederholfrequenz der Schläge des Schlagteils auf den Prallkörper und der daraus resultierenden eingekoppelten Druckwellen.

Im Übrigen erlauben viele Geräte den Austausch von Prallkörpern gegen hinsichtlich Geometrie und/oder Masse abweichende andere.

Der vorliegenden Erfindung liegt das technische Problem zugrunde, ein hinsichtlich des Prallkörpers verbessertes Gerät und insbesondere ein vorteilhaftes Verfahren zur Vorbereitung des Geräts auf die Behandlung anzugeben.

Die Erfindung richtet sich auf ein Verfahren zur regelmäßigen Vorbereitung eines Geräts zur Behandlung des menschlichen oder tierischen Körpers durch mechanische Druckwellen auf eine solche Behandlung, welches Gerät aufweist:
ein bewegbares Schlagteil und
einen Prallkörper, der austauschbar ist,
wobei die Druckwellen durch Beschleunigen und Aufprallen des Schlagteils auf den Prallkörper erzeugbar und durch extrakorporales Aufsetzen auf die Körperoberfläche einkoppelbar sind,
**dadurch gekennzeichnet, dass** der Prallkörper vor jeder Behandlung eines weiteren Patienten gegen einen unbenutzten Prallkörper ausgetauscht wird,
sowie auf ein entsprechendes Gerät, das mit einem Einmalprallkörper ausgestattet ist.

Der erfindungsgemäße Einsatz von Einmalprallkörpern bedeutet den Austausch des Prallkörpers mindestens mit der Behandlung jedes neuen Patienten, Er bedeutet nicht zwingend den Austausch des Prallkörpers zwischen unmittelbar aufeinander folgenden Behandlungen desselben Patienten, also ohne dazwischenliegende Behandlung eines anderen Patienten.

Durch diesen Austausch erübrigt sich eine gründliche Reinigung und ggf. Desinfektion oder Sterilisierung eines bereits für einen anderen Patienten verwendeten Prallkörpers zwischen den Behandlungen. Dies spart nicht nur Zeit und Reinigungs- und Sterilisiermittel; es müssen auch keine entsprechenden Arbeitsplätze und Sterilisiergeräte vorgehalten werden.

Zudem werden die Prallkörper in der Praxis häufig mit pastösen Mitteln bestrichen, um den Kontakt zur Haut und den Druckwellenübergang zu verbessern. Solche Pasten können nicht nur in Kontakt mit dem Prallkörper selbst sondern auch beispielsweise mit einer zu seiner Montage dienenden Abdeckkappe oder anderen Geräteteilen kommen. Auf die Dauer besteht die Gefahr, dass das Gerät stellenweise verklebt oder zugesetzt wird. Aus diesem Grund ist es von Vorteil, den Prallkörper möglichst häufig auszubauen. Wenn dieser Aufwand einmal betrieben wird, ist damit schon ein wesentlicher Teil des durch die Verwendung von Einmalprallkörpern verursachten Mehraufwandes betrieben.

Überdies zeigen auch für einen längeren Betrieb ausgelegte Prallkörper Ermüdungserscheinungen und sind Ausfälle, insbesondere durch Materialbrüche oder Risse, wegen der damit verbundenen Verletzungsgefahr unbedingt zu vermeiden. Die Verwendung von neuen Einmalprallkörpern garantiert in besonders einfacher Weise, dass keine unzulässig große Belastung von Prallkörpern auftritt. Sie erübrigt damit eine vergleichsweise komplexere Lebensdauerüberwachung.

Zudem können bei der Herstellung der Prallkörper, insbesondere was das Material betrifft, deutliche Einsparungen vorgenommen werden, eben weil die geforderten Lebensdauern vergleichsweise sehr kurz sind. Dies betrifft insbesondere verschiedene bevorzugte Materialien, auf die im Folgenden noch näher eingegangen wird, aber auch die Herstellverfahren.

Schließlich sind durch Verwendung von Einmalprallkörpern besonders hohe Reinheits- und Hygienestandards möglich. Die Prallkörper können örtlich unabhängig von Ihrem Einsatzort sterilisiert und verpackt werden und am Einsatzort muss diesbezüglich keinerlei Aufwand mehr betrieben werden.

Benutzte Prallkörper können weggeworfen werden oder, abhängig vom Materialwert, auch recycelt werden.

Die Erfindung richtet sich also sowohl auf (durchaus bevorzugte) Varianten, bei denen die Prallkörper nach Gebrauch weggeworfen werden, also keiner weiteren Verwendung als Prallkörper zugeführt werden. Andererseits richtet sich die Erfindung auch auf andere Varianten, bei denen die Prallkörper gesammelt und zur Rohstoffwiedergewinnung genutzt werden (insbesondere bei Metallen), aber nicht als Prallkörper erhalten bleiben. Schließlich richtet sich die Erfindung auch auf solche Ausführungen, bei denen die Prallkörper als solche erhalten bleiben, aber (außerhalb der Klinik oder Praxis der Anwendung oder auch innerhalb) vollständig gereinigt und ggf. sterilisiert werden, um dann wieder als Prallkörper eingesetzt zu werden. Im letztgenannten Fall fallen die oben erwähnten Vorteile hinsichtlich des Wegfalls der Notwendigkeit einer Lebensdauerüberwachung natürlich nicht ins Gewicht. Hier ist durch Austausch des Prallkörpers vor jeder Patientenbehandlung aber dennoch ein Hygienegewinn möglich und wird eine hartnäckige Verschmutzung anderer Gerateteile besser verhindert als bei dauerhaftem Gebrauch desselben Prallkörpers.

Bevorzugte Materialien sind zum einen Kunststoffe, zum zweiten Metalle und zum dritten gesinterte Keramiken. Günstige Kunststoffe können beispielsweise Polykarbonat oder PEEK (Polyetheretherketon) sein. Kunststoffe, insbesondere Thermoplaste, lassen sich vor allem durch Spritzgussverfahren besonders kostengünstig und trotzdem formtreu herstellen. Bevorzugte Metalle sind insbesondere Aluminium oder Edelstahl. Sie können nicht nur als Drehteile hergestellt werden, sondern auch durch Sinterverfahren oder Gussverfahren, insbesondere Hochdruckgussverfahren. Als Metalle sind hier auch Werkstoffe gemeint, die sich durch Formen eines Gemischs aus Metallpulvern und eines Kunststoff als Matrix (oder eines Bindermaterials) und nachfolgendes Ausbacken herstellen lassen. Diese sinterähnlichen Materialien sind letztlich feste Metallpulvergefüge.

Im Übrigen sind gesinterte Keramiken günstig, insbesondere und beispielsweise Zirkonoxid, Siliziumoxid und Siliciumnitrid.

Kunststoffe und Keramiken haben in vielen Fällen eine gute Biokompatibilität. Daneben zeigen sie im Vergleich zu Metallen in der Regel eine deutlich geringere Wärmeleitfähigkeit. Der Patient fühlt jedenfalls bei direkter Berührung der Haut subjektiv ein wärmeres und damit angenehmeres, weniger fremd wirkendes Teil.

Zudem sind viele Keramiken und Kunststoffe deutlich leichter als übliche Metalle, insbesondere rostfreier Stahl. Dies hat den Vorteil, dass das aus baulichen Gründen üblicherweise kleinere Schlagteil bei einer üblichen metallischen Ausführung einen kleineren Masseunterschied zu dem Prallkörper aufweist und damit die Stoßbedingungen im Sinne eines möglichst großen Impuls- und Energieübergangs verbessert werden. Zudem können auch etwas größere Auslenkungen des Prallkörpers erzielt werden, was von interesse sein kann.

Schließlich zeigen viele Keramiken auch günstige akustische impedanzen, die sich von den typischen akustischen impedanzen von Körpergewebe weniger unterscheiden als Metalle, etwa rostfreier Stahl. Dies ist im Wesentlichen Ergebnis der geringeren Dichte, aber auch von der Schallgeschwindigkeit abhängig. Auch Kunststoffe haben in der Regel günstigere akustische impedanzen für die vorliegenden Anwendungen als Metalle, sodass das Argument hier analog gilt.

Unter Keramik wird hier ein aus anorganischen feinkörnigen Rohstoffen gewonnenes Material verstanden, das gesintert ist, also einen Temperschritt erfahren hat. Besonders bevorzugt sind Oxide, insbesondere Metalloxide. Carbide, insbesondere Metallcarbide und Nitride, sowie Mischungen daraus. In Betracht kommen beispielsweise Zirkonoxid, Siliziumoxid oder Siliziumnitrid. Bevorzugt liegt der relative Anteil dieser Materialien bei mindestens 80 Gew.-%, besser 85 Gew.-%, 90 Gew.-% bzw. 95 Gew.-%.

Andere Bestandteile können aber ebenfalls enthalten sein. Insbesondere können in einem gewissen Anteil von maximal 20 Gew.-%, besser maximal 15 Gew.-% bzw. 10 Gew.-% bzw. 5 Gew.-% metallische Anteile vorhanden sein, also metallische Partikel oder Pulver. Aus dem Bereich der Pulvermetallurgie sind für Metalle auch ähnliche Verarbeitungstechniken wie die Keramiksinterung bekannt. Bei den genannten Werten werden die positiven Eigenschaften der Keramik durch diese metallischen Zusätze nicht wesentlich verschlechtert. Im günstigsten Fall sind allerdings keine metallischen Anteile vorhanden.

Schließlich sind bestimmte Parameter der verwendeten Keramik bevorzugt, insbesondere eine relativ geringe Dichte von vorzugsweise unter 6 g/cm³, besonders bevorzugter Welse unter 5 g/cm³ und noch bevorzugter Weise unter 4 g/cm³. Eine niedrige Dichte verringert die Masse des Prallkörpers und damit die (günstigerweise mit der Hand zu handhabende) Masse des mobilen Teils der Vorrichtung. Sie reduziert auch die bereits erwähnte akustische impedanz in günstiger Weise. Schließlich ermöglicht sie eine gewisse Baugröße des Prallkörpers ohne zu große Massenunterschiede zwischen Prallkörper und Schlagteil.

Bevorzugt ist ferner eine sog. Schlagzähigkeit des Keramikmaterials von mindestens 3 MPam, besser 4 MPam und noch günstiger 5 MPam oder mehr. Diese Größe bestimmt, mit welcher Heftigkeit das Schlagteil auf den Prallkörper auftreffen kann, ohne den Prallkörper selbst zu gefährden.

Schließlich sind relativ harte Materialien bevorzugt, insbesondere solche mit einer größeren Druckfestigkeit von über 2000 MPa.

Keramik bietet schließlich die Möglichkeit, das Material unproblematisch und weitgehend beliebig zu färben. Neben dekorativen Gesichtspunkten kann dies in vorteilhafterweise dazu genutzt werden, unterschiedliche Prallkörpertypen leicht von einander unterscheidbar zu machen. Bei vielen Anwendungsfällen stehen dem Nutzer hinsichtlich Masse, Form oder Material unterschiedliche Prallkörper zur Verfügung. Beispielsweise können auch fokussierende Formen Verwendung finden, etwa mit gewölbter Austrittsfläche oder als Rotationsellipsoid, vgl. DE 102 2007 013 288. Bei einer farblichen Kodierung sind Fehler unwahrscheinlicher als bei einer alphanumerischen Bezeichnung (die natürlich zusätzlich vorhanden sein kann).

Hinsichtlich der Herstellung des Keramikprallkörpers sind solche Sinterverfahren bevorzugt, bei denen auf den Rohling bzw. werdenden Prallkörper Druck ausgeübt wird. Dies kann vor und/oder während des Temperschritts erfolgen. Insbesondere kann eine isostatische Nachverdichtung unter Hitzeeinwirkung erfolgen.

Abhängig von der Belastbarkeit des Materials und von den Leistungsdaten des Gerätes, in dem ein Prallkörper verwendet wird, sind für Prallkörper sinnvoller Weise maximale Pulszahlen vorgegeben. Durch die Erfindung kann sichergestellt werden, dass der Benutzer nicht durch Vergessen oder Vermeidung des rechtzeitigen Einsetzens eines neuen Prallkörpers die zugelassene Nutzungsdauer eines Prallkörpers überschreitet, einfach indem grundsätzlich vor jeder Neubehandlung ein Austausch erfolgt.

Typische Pulszahlen für Behandlungen in der Schmerztherapie liegen in der Größenordnung von einigen 1.000, etwa um die 2.000 Pulsen bei kleineren und bis zu größenordnungsmäßig 8.000 Pulsen bei großen Muskeln. In anderen Anwendungsbereichen, insbesondere in der ästhetischen Dermatologie, etwa bei der Cellulitisbehandlung, tauchen deutlich größere Pulszahlen auf, die über 10.000 und bis zu 20.000 pro Patient betragen können, wobei mehrere Behandlungen bei einem Patienten zusammengefasst sein können, etwa an beiden Oberschenkeln. Insbesondere ist die Erfindung somit bei Verwendungen bevorzugt, bei denen recht hohe Pulszahlen pro Patient auftreten, insbesondere bei Pulszahlen pro Patient von über 5.000, vorzugsweise über 8.000, besonders bevorzugterweise über 12.000. Hier werden möglicherweise schon nach wenigen Patienten Lebensdauergrenzen erreicht wenn kein Austausch erfolgt. Im Übrigen kann es günstig und bevorzugt sein, bei einem erfindungsgemäßen Austausch des Prallkörpers die zugehörige Applikatorkappe, also die zu seiner Montage dienende Kappe (im Ausführungsbeispiel ein Ring mit Gewinde) mit auszutauschen. Oft ist diese Applikatorkappe von Verschmutzungen mit betroffen und verursacht keinen wesentlichen zusätzlichen Kostenaufwand.

Bei einer weiteren Ausgestaltung der Erfindung soll der verwendete Prallkörper von dem Gerät erkannt werden. Dies kann seinen Typ oder bestimmte technische Eigenschaften, also in irgendeiner Weise eine Prallkörpergattung, aber auch eine Seriennummer oder eine ähnliche individuelle Kennzeichnung betreffen. Bei der Erkennung der Prallkörpergattung kann es beispielsweise um die Unterscheidung von Applikatoren hinsichtlich ihrer geometrischen Form, insbesondere ihrer Austrittsgrenzflächenwolbung (flach, konvex oder konkav und in welchem Maße gewölbt), oder hinsichtlich ihrer Materialien (Härte, Schailleitungseigenschaften) oder auch ihrer Massen gehen. Bei der Erkennung eines individuellen Prallkörpers, der also hinsichtlich seiner individualität von einem bautypgleichen unterschieden werden kann, geht es im Rahmen dieser Erfindung primär um die Sicherstellung der Verwendung eines wirklich neuen Prallkörpers, also die Sicherstellung des erfolgten Austauschs vor der Behandlung des nächsten Patienten.

Schließlich kann das Gerät anhand einer Prallkörpererkennung auch prüfen, ob der eingesetzte Prallkörper überhaupt für das Gerät geeignet ist, und ggf. den Betrieb verweigern oder eine Warnung abgeben.

Grundsätzlich kann die Erfindung ausgeführt werden, ohne den Prallkörper hierzu in besonderer Weise auszustatten. Beispielsweise könnte die Erkennungseinrichtung unterschiedliche magnetische Eigenschaften metallischer Prallkörper mit ausreichend unterschiedlichen Formen und/oder Metallmaterialien ermitteln und unterscheiden, etwa über eine Induktivitätsmessung, also durch Einsatz einer in dem übrigen Behandlungsgerät angebrachten elektromagnetischen Spule. Allgemeiner gesprochen können elektromagnetische Eigenschaften wie die elektrische Leitfähigkeit des Prallkörpers zur Erkennung dienen. Dies gilt natürlich auch für andere Eigenschaften, die der Prallkörper ohnehin hat, etwa für die Masse.

Bevorzugt ist allerdings eine Markierung des Prallkörpers, also eine zu dem Zweck der Erkennung vorgesehene Gestaltung oder Einrichtung. Dabei soll im Folgenden eine Befestigungseinrichtung für den Prallkörper, etwa die Applikatorkappe der Ausführungsbeispiele, im Sinne der Markierung dann als Prallkörperteil betrachtet werden, wenn sie beim Austauschen des Prallkörpers mit diesem gemeinsam ausgetauscht wird, ihm also zugeordnet ist. Hinsichtlich der Funktion der Erkennung läuft dann eine Erkennung einer dem Prallkörper zugeordneten Befestigungseinrichtung und eine Markierung des Prallkörpers selbst auf das Gleiche hinaus. In diesem Sinn kann der Begriff des Prallkörpers im Folgenden also auch eine Einheit aus Prallkörper und Befestigungseinrichtung meinen, wobei die Markierung in der Befestigungseinrichtung liegen kann.

Eine erfindungsgemäße Gruppe von Markierungen verfügt über zumindest einen elektrischen Kontakt, etwa um einen elektrischen Widerstand des Prallkörpers oder an dem Prallkörper messen zu können. Dieser Widerstand kann beispielsweise ein zusätzlicher Leiter sein, der außen an dem Prallkörper angebracht ist, etwa ein über einen Abschnitt gelegter Leiterstreifen. Im Ausführungsbeispiel wird ein ringförmiger Leiterstreifen dargestellt, der über einen Umfangsabschnitt des Prallkörpers verläuft.

Eine andere Gruppe von Markierungen wird über Licht im weitesten Sinn ausgelesen, also einschließlich Infrarotstrahlung. Beispielsweise kann über Licht ein Code ausgelesen werden, der aus einer bestimmten Folge von mehr oder weniger reflektierenden Flächen besteht, im Falle von sichtbarem Licht etwa ein aus hellen und dunklen Flächen bestehender Streifen, insbesondere ein sog. Barcode.

Insbesondere bei der Erkennung von Markierungen über Licht, aber auch unabhängig davon, kann es notwendig oder gewünscht sein, den Prallkörper in einer bestimmten Orientierung hinsichtlich seiner Längsachse einzubauen. Zur Vorgabe einer solchen Orientierung können Formschlusselemente dienen, also beispielsweise vorspringende Teile, die in an dem Prallkörper eingebrachte Nuten eingreifen. Dann kann der Prallkörper nur in einer bestimmten gewünschten Orientierungen (oder in einer Mehrzahl jeweils geeigneter Orientierungen) angebracht werden.

Eine andere Möglichkeit besteht darin, einen Magneten, etwa in dem Prallkörper, und einen dazu passenden Magnetsensor im übrigen Gerät vorzusehen, oder auch umgekehrt.

Zur Auslesung über Licht, also zur optischen Auslesung, kann ein optischer Leiter vorgesehen sein, der vorzugsweise zwischen einer Außenhülse des Gerätes und einem innenrohr, das zum Führen des Schlagteils dient, verlaufen kann. Zur Veranschaulichung wird auf die Ausführungsbeispiele verwiesen.

Eine weitere Möglichkeit zur Erkennung von Markierungen besteht in der Verwendung von elektromagnetischen Wellen, etwa Radiofrequenzwellen. Hierzu kann die Markierung einen Transponder aufweisen und die Erkennungseinrichtung eine Empfangs/Sendespule, die den Typ des Transponders feststellen kann. Die Empfangs/Sendespule kann beispielsweise zwischen dem bereits erwähnten innenrohr und der bereits erwähnten Außenhülse angeordnet sein, wozu wieder auf die Ausführungsbeispiele verwiesen wird. Die Empfangs/Sendespule kann aber auch in einem Basisgerät untergebracht sein, das zur Versorgung eines mobilen Handgerätes dient und mit dem Handgerät über eine Leitung verbunden ist. In diesem Fall kann es notwendig sein, den Prallkörper vor der Montage oder das Handgerät mit dem bereits darin montierten Prallkörper in die Nähe des Basisgerätes zu bringen, um die Erkennung der Markierung zu ermöglichen, also das Auslesen des Transponders.

Die Prallkörpererkennung kann insbesondere dazu führen, dass ein erkannter Typ des Prallkörpers angezeigt wird. Damit hat ein Nutzer die Möglichkeit, den Prallkörpertyp zu kontrollieren und/oder bestimmte Bedienungsparameter. Geräteparameter oder andere Umstände der Nutzung darauf abzustimmen.

Überdies kann das Gerät vorzugsweise selbstständig zur Einstellung geeigneter Betriebsparameter für den erkannten Prallkörpertyp ausgelegt sein bzw. dazu, eingestellte Betriebsparameter in dieser Hinsicht zu kontrollieren (und bei Widersprüchen ein Signal abzugeben oder den Betrieb zu verweigem).

Schließlich ist die Verwendung der Vorrichtung gerade bei der Behandlung von Körperweichgewebe, beispielsweise Muskeln oder Sehnen, bevorzugt. Dies schließt die Behandlung knochennaher Bereiche und eine Stoßwellenakupunktur ein. Typische Indikationen sind Ansatztendinosen und andere Anwendungen in Orthopädie und Chirurgie wie Kalkschultern, Fersenschmerzen, Pseudarthrosen, aber auch Muskelzerrungen. Weitere Indikationen gibt es in der Neurologie, etwa die Verbesserung der Motorik nach Schlaganfällen, die Behandlung von Spasmen nach Traumen sowie Poly-Neuropathien. In der Urologie können etwa chronische Beckenbodenschmerzen behandelt werden; in der Angiologie / Dermatologie und Chirurgie außerdem Narben oder Hautverbrennungen behandelt sowie eine Verbesserung der Wundheilung erzielt werden.

Die Erfindung wird anhand einiger Ausführungsbeispiele näher erläutert, wobei die einzelnen Merkmale für alle genannten Anspruchskategorien und auch in anderen als den dargestellten Kombinationen erfindungswesentlich sein können.
- Figur 1: zeigt ein erfindungsgemäßes Gerät im Längsschnitt, wobei Ein- zelheiten der Erfindung in Figur 1 nicht dargestellt sind.
- Figuren 2 - 9: zeigen jeweils einen Ausschnitt aus Figur 1 mit zusätzlich darge- stellten Erkennungseinrichtungen und/oder Markierungen.

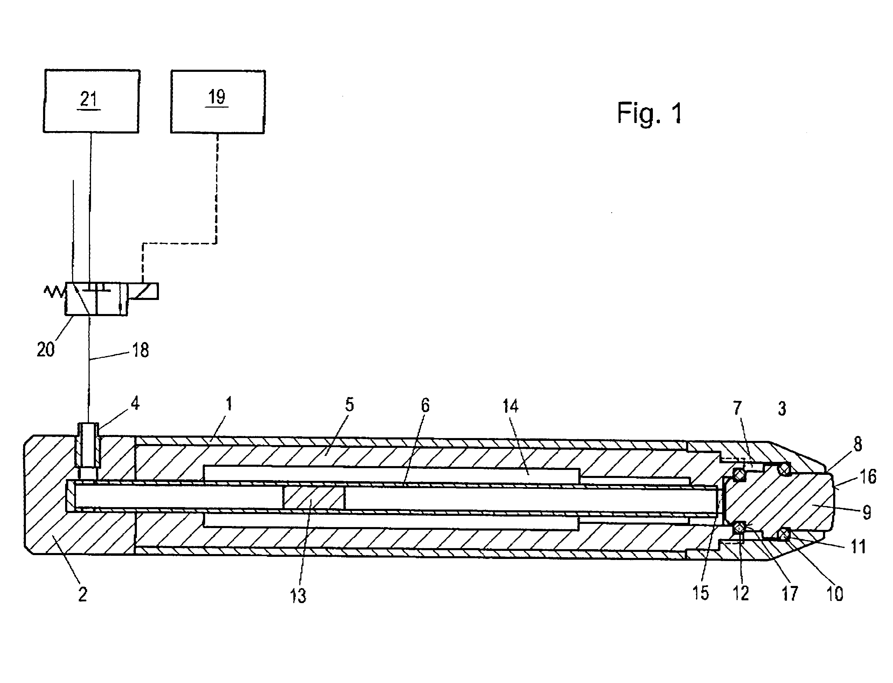

In Figur 1 ist in einem Schnitt entlang einer Längsachse eine erfindungsgemäße Vorrichtung zur Einkopplung von fokussierten mechanischen Druckwellen in beispielsweise den menschlichen Körper dargestellt. Ein Rohrstück bildet eine Außenhülse 1, die von einer in der Anwendung körperfernen Zuluftkappe 2 und einer in der Anwendung körperzugewandten Applikatorkappe 3 jeweils endseitig abgeschlossen ist.

Die Zuluftkappe 2 enthält einen Druckluftanschluss 4 für eine pneumatische Versorgung. In an sich bekannter Weise ist an diesen Druckluftanschluss 4 über eine pneumatische Versorgungsleitung ein von einer Ansteuereinheit 19 gesteuertes Ventil 20, insbesondere Magnetventil, angeschlossen, das in einem gleich bleibenden iterativen Takt zwischen etwa 1 Hz und 50 Hz Druckluftpulse über den Druckluftanschluss einkoppelt.

Die Vorrichtung ist als mit der Hand einer Bedienungsperson zu haltendes Gerät ausgebildet, das über die erwähnte Pneumatikleitung 18 an eine Basisstation mit der Ansteuereinheit 19 und dem Kompressor 21 angeschlossen ist und auf den Patienten manuell aufgesetzt werden kann. Es dient zur Behandlung von Weichgewebe, insbesondere Muskeln.

Die Einzelheiten der Pneumatikversorgung sind nicht Gegenstand dieser Erfindung und dem Fachmann aus dem Stand der Technik geläufig. Vorzugsweise ist die Frequenz einstellbar. Der iterative Betrieb kann komplizierter als mit einer einfachen gleich bleibenden Wiederholung von Pulsen einer bestimmten Frequenz erfolgen, insbesondere auch mit einer Mehrzahl in relativ kurzem Zeitabstand, also mit einer relativ hohen Frequenz, aufeinander folgenden Schlägen, wobei Gruppen von Schlägen in diesem kürzeren Zeitabstand durch etwas größere Zeitabstände voneinander getrennt sind. Die Einzelheiten hierzu sind nicht Gegenstand der vorliegenden Erfindung, können aber damit kombiniert sein.

In der Außenhülse 1 ist über einen Einsatz 5 ein Führungsrohr 6 gehalten, dessen bei der Anwendung körperfernes Ende in der Zuluftkappe 2 endet und dort mit dem Druckluftanschluss 4 kommuniziert. Das in der Anwendung körperseitige Ende des Führungsrohres 6 endet in einem Teil des Einsatzes 5, der in die Applikatorkappe 3 hineinragt, und zwar kurz vor dem dortigen Ende des Einsatzes 5 und einem Innenraum 7 in der Applikatorkappe 3.

In dem Innenraum 7, der mit zwei radialen Schultern in eine in der Anwendung körperseitige Applikatoröffnung 8 übergeht, ist ein Prallkörper 9 aufgenommen. Dieser stützt sich über einen O-Ring 10 aus einem Elastomer an einer der radialen Schultern ab und weist hierzu einen Flansch 11 auf. Ein zur körperfemen Seite gerichtetes Ende 15 des Prallkörpers 9 stützt sich über einen weiteren O-Ring 12 an dem Einsatz 5 ab, und zwar an einer das bereits erwähnte Ende des Einsatzes 5 umgebenden Stirnfläche. Dabei liegt der O-Ring 12 zwischen dieser Stirnfläche und einem Flansch 17 bzw. einer Schulter des Prallkörpers 9. Die Applikatoröffnung 8 dient dabei zu einer in der Längsrichtung verschiebbaren Führung des Prallkörpers 9 und fixiert diesen quer zur Längsrichtung. Die Axialverschiebbarkeit ist durch die Nachgiebigkeit der Elastomerringe 10 und 12 begrenzt und liegt bei in Luft betriebener Vorrichtung relativ zur Restvorrichtung deutlich über 0,6 mm.

Auf die Merkmale des Prallkörpers 9, der hier gleichzeitig den auf die Haut aufzusetzenden Applikator bildet, wird im Folgenden noch näher eingegangen. Er ist durch Abschrauben der Applikatorkappe 3 austauschbar.

In dem angrenzenden Bereich des Führungsrohres 6 ist ein in Figur 1 mit dem Prallkörper 9 in Kontakt stehendes Schlagteil 13 eingesetzt. Dieses passt (in Bezug auf das Führungsrohr und die im Wesentlichen zylindrische Geometrie des Schlagteils 13) radial mit geringem Spiel. Das Schlagteil 13 kann durch Druckunterschiede der Luftsäule in dem Führungsrohr 6 vor und hinter ihm (d. h. in Figur 1 rechts und links des Schlagteils 13) in dem Führungsrohr hin- und herbewegt werden und insbesondere auf den Prallkörper 9 zu beschleunigt werden. Hierzu wird es aus einer Ausgangsposition (nicht gezeigt) in Figur 1 links durch einen Druckluftstoß durch den Druckluftanschluss 4 beschleunigt und trifft mit seiner dem Prallkörper 9 zugewandten Frontfläche (in Figur 1 der Übersichtlichkeit halber nicht bezeichnet) auf den Prallkörper 9 auf.

Die Rückbewegung des Schlagteils 13 erfolgt zusätzlich zu einem Zurückprallen nach der Kollision durch ein Rückströmen der Luft aus einer das Führungsrohr 6 innerhalb des Einsatzes 5 umgebenden Staukammer 14. In diese wird die Luft bei der Beschleunigung des Schlagteils 13 in Richtung zu dem Prallkörper 9 verdrängt und damit dort komprimiert. Wenn das Magnetventil 20 in der Pneumatikzuleitung 18 des Druckluftanschlusses 4 den Druck wegschaltet, wird das Schlagteil 13 damit in die Ausgangsstellung zurückbewegt. Dies kann natürlich auch durch eine zusätzliche oder alternative Druckbeaufschlagung der Staukammer 14 oder eines anderen Luftvolumens körperseitig von dem Schlagteil 13 erfolgen. Das in der Anwendung körperferne Ende des Führungsrohres 6 endet in einem Magnethalter 17 für das Schlagteil 13.

Der Prallkörper 9 hat eine rotationssymmetrische Zylinderform und ist in axialer Richtung durch die Eintrittsfläche 15 und die etwas konvexe Austrittsfläche 16 begrenzt. Der Außenmantel weist die bereits beschriebenen flanschartigen Strukturen 11 und 17 auf, die Anlageschultem für die O-Ringe 10 und 12 bilden. Im Übrigen ist ein austrittsseitiger Teil der Zylindergeometrie mit konstantem Radius gestaltet und damit in der Öffnung 8 axial verschiebbar.

Prallkörper wie der hier gezeichnete Prallkörper 9 können sich hinsichtlich Form, Material und Lagerung erheblich unterschieden. So gibt es unterschiedliche fokussierende und nicht fokussierende Formen, vergleiche etwa auch die Rotationsellipsoidform in der DE 10 2007 013 288, und verschiedene gewölbte Austrittsflächen im Stand der Technik. Ferner kommen verschiedene Materialien in Betracht, etwa Edelstahl, Titan, und verschiedene Keramiken wie Siliziumnitrid sowie Kunststoffe. Schließlich können verschiedene Prallkörper unterschiedlich hart gelagert sein und dabei unterschiedliche Hübe ausführen, also eine unterschiedlich lange makroskopische Bewegung bei der Einkopplung der Druckwelle durchführen.

Verschiedene Ausgestaltungen in diesem Sinn sind jeweils für bestimmte Anwendungen besonders gut geeignet, etwa für Akupunktur, Sehnenansatzbehandlungen, Muskeibehandlungen, Triggerpunktbehandlungen etc.. Hierbei werden verschiedene Parameter für beispielsweise den Antriebsdruck oder die Pulsfrequenz eingesetzt, die von dem verwendeten Prallkörper und der Indikation abhängen. Im Sinne einer sinnvollen Systemsteuerung ergibt sich daher ein erheblicher Vorteil, wenn eine automatische Erkennung des Prallkörpertyps durchgeführt wird.

Bei einer Variante besteht der Prallkörper 9 aus gesinterter Keramik, und zwar einem verdichtetem Siliciumnitrid-Material (Si₃N₄). Es handelt sich um polykristallines Material mit tetragonaler Kristallstruktur, das sich als erstaunlich schlagzäh und fest erwiesen hat. Quantitativ kann die Schlagzähigkeit angegeben werden mit 6,5 - 7 MPam bei einer Druckfestigkeit von etwa 3000 Mpa.

Das Material ist relativ leicht, verfügt nämlich über eine Dichte von 3,2 g/cm³. Da die Schallgeschwindigkeit für longitudinale Druckwellen zwar höher als in rostfreiem Stahl liegt, aber nicht zu hoch, ergibt sich eine um etwa 20 % - 25 % reduzierte akustische impedanz, die dementsprechend näher an der akustischen Impedanz vom Körpergewebe liegt. Die Einkopplung der Druckwellen in das Körpergewebe erfolgt damit noch etwas günstiger als bei konventionellen Prallkörpern.

Das Material hat ferner eine Wärmeleitfähigkeit in der Größenordnung von 20 W/mK und wirkt damit sensorisch weniger kalt als Stahl. Es ist auf Biokompatibilität getestet.

Hier kann beispielhaft verwiesen werden auf biologische Versuche, über die berichtet wird in "Biokompatibilität von Siliziumnitrit-Keramik in der Zellkultur. Eine vergleichende fluoreszenzmikroskopische und rasterelektronenmikroskopische Untersuchung", Laryngo-Rhino-Otol 2004, 83: 845-851, auch in Thieme-connect des Georg Thieme Verlags und Thieme Medical Publishers, Inc.

Schließlich kann der Prallkörper 9 unproblematisch gefärbt werden (etwa durch Zusatz von farbigen Metallionen wie Co), sodass unterschiedlich geformte und/oder unterschiedlich schwere Prallkörper zur Variation verschiedener Behandlungsparameter, insbesondere des Hubes, der Größe der Austrittsfläche 16 oder ihrer Form usw. durch verschiedene Farben gekennzeichnet und verwechslungssicher montiert werden können. Dazu ist die Applikatorkappe einfach abschraubbar.

Alternativ können PEEK- , Polykarbonat- oder auch Aluminiumprallkörper 9 verwendet werden, wie weiter oben schon erläutert. Auch konventioneller Edelstahl ist geeignet.

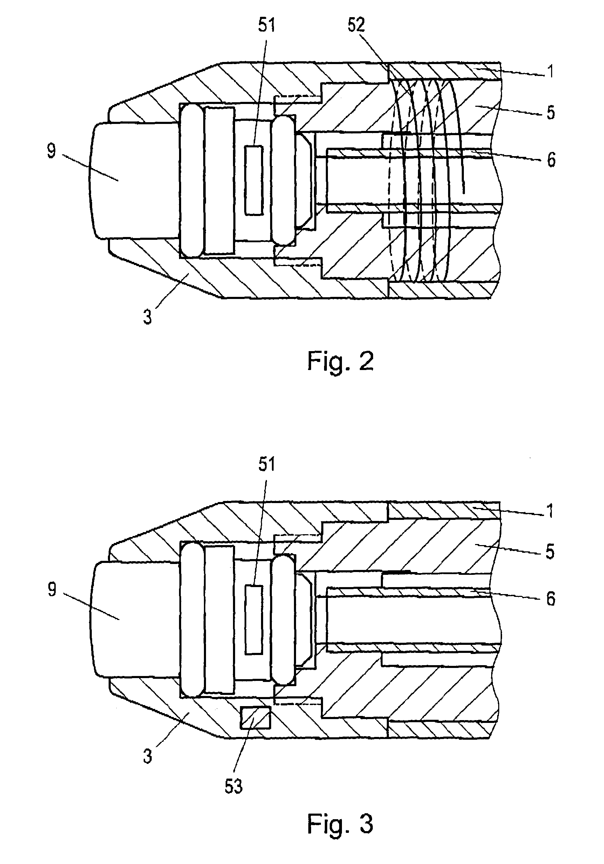

Figur 2 zeigte ein erste Variante mit Prallkörpererkennung als (seitenverkehrten) Ausschnitt aus Figur 1 mit zusätzlich eingezeichneten Einzelheiten zur Erfindung. Figur 2 zeigt einen sogenannten RFID-Transponder 51, der auf dem Prallkörper 9 angebracht ist. Mit 52 ist eine nur symbolisch angedeutete Empfangs/Sendespule in dem Handteil aus Figur 1 dargestellt. Diese ist zwischen der Außenhülse 1 und dem Einsatz 5 und dabei möglichst nah an dem Prallkörper 9 und dem daran montierten RFID-Transponder 51, also möglichst weit links in Figur 2, montiert. Man erkennt in der Figur, dass beim Abnehmen der Applikatorkappe 3 der Bereich, in dem die Empfangs/Sendespule 52 angebracht ist, dennoch nicht geöffnet wird und diese insoweit nicht gefährdet ist. Durch die Nähe erleichtert sich die Erkennung des RFID-Transponders 51. Insbesondere werden Kopplungen zu anderen Prallkörpern, die beispielsweise auf einem Tisch im Behandlungszimmer in der Nähe des Handstücks liegen könnten, in dieser Weise möglichst schwach relativ zu der Kopplung an den dargestellten RFID-Transponder 51 gehalten.

RFID-Erkennungssysteme sind an sich bekannt. Über ein elektromagnetisches Hochfrequenzfeld mit typischerweise 13,56 MHz wird der RFID-Transponder 51 mit Energie versorgt und ausgelesen. Da hier nur kurze Reichweiten erforderlich sind, benötigt das System nur geringe Leistungen. Ferner kann der RFID-Transponder 51 sehr klein sein und beeinträchtigt die Eigenschaften des Prallkörpers 9 nicht.

In Figur 3 ist in analoger Weise eine zweite Variante zur Erkennung dargestellt. Auch hier ist ein RFID-Transponder 51 vorgesehen, wobei jedoch die Empfangs/Sendespule in dem nicht dargestellten Basisgerät, vgl. Figur 1, enthalten ist. Bei solchen Ausgestaltungen der Erfindung muss der Nutzer also grundsätzlich mit dem noch nicht eingebauten einzusetzenden Prallkörper 9 oder auch dem eingebauten Prallkörper 9, also dem körperseitigen Ende des Handstücks, in die Nähe des Basisgeräts kommen, um eine Erkennung zu ermöglichen. Bei diesem Ausführungsbeispiel ist allerdings eine zusätzliche Verbesserung insoweit vorgesehen, als die Gefahr ausgeschlossen werden soll, dass versehentlich ein in der Nähe des Basisgerätes befindlicher Prallkörper, der aber gar nicht zum Einsatz vorgesehen ist, erkannt wird. Dazu verfügt dieses Ausführungsbeispiel über einen Permanentmagneten 53 in der Applikatorkappe 3, die den Prallkörper 9 an dem Handstück hält. Der RFID-Transponders 51 kann die Anwesenheit des Permanentmagneten 53 erkennen und damit den eingebauten Zustand des Prallkörpers 9 von einem nicht eingebauten Zustand unterscheiden. Der RFID-Transponder wird gewissermaßen durch die Detektion des Permanentmagneten 53 freigeschaltet.

In diesem Zusammenhang kann es von Vorteil sein, den RFID-Transponder 51 nicht, wie gezeigt, von dem Permanentmagneten unabhängig an dem Prallkörper 9 zu montieren, sondern an dem Randbereich, der eine möglichst große räumliche Nähe zwischen dem Permanentmagneten 53 und dem RFID-Transponder 51 gewährleistet. Dazu kann ein nicht dargestellter Formschluss dargestellt sein, etwa eine Nut in dem Prallkörper 9 und eine passende Nase in der Applikatorkappe 3. um bei der Montage des Prallkörpers 9 die drehsinnrichtige Position zu gewährleisten und eine versehentliche Anordnung des RFID-Transponders 51 in einer anderen als der dem Permanentmagneten 53 angenäherten Winkelposition zu verhindern.

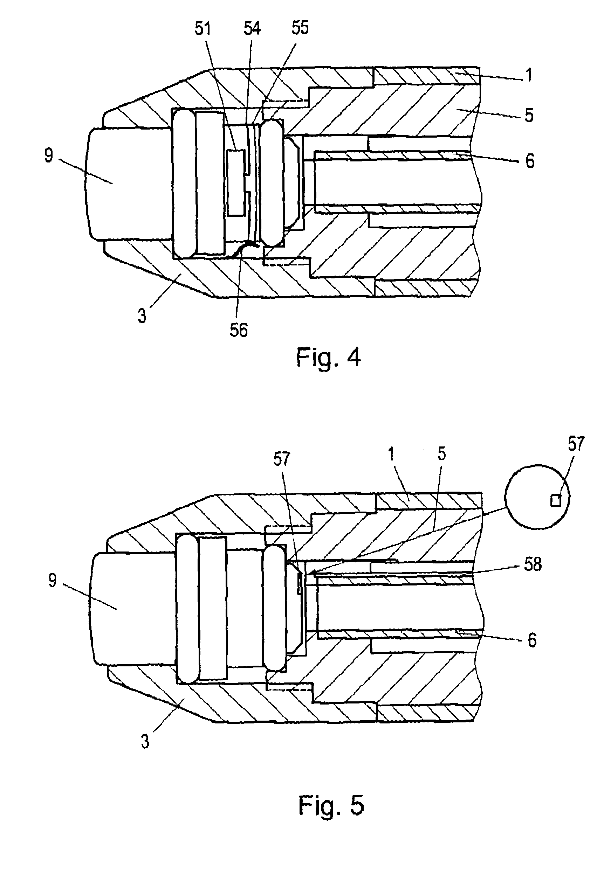

Figur 4 zeigt wieder den RFID-Transponder 51 aus den Figuren 2 und 3. Dieser ist hier allerdings über Leitungen an zwei Ringelektroden 54 und 55 angeschlossen, die über einen in Figur 4 im unteren Bereich dargestellten Federkontakt 56 in der Applikatorkappe 3 kontaktiert werden können. Der Federkontakt 56 kann die beiden Ringelektroden 54 und 55 kurzschließen und damit in einer ähnlichen Weise wie in Figur 3 den RFID-Transponder 51 des montierten Prallkörpers 9 von anderen unterscheidbar machen. Im Übrigen gelten die Erläuterungen zu Figur 3.

Das nächste Ausführungsbeispiel in Figur 5 verwendet eine optisch erkennbare Markierung des Prallkörpers 9, nämlich einen zweidimensionalen Barcode 57. Dieser ist an einer körperabgewandten Stirnfläche des Prallkörpers 9 exzentrisch angebracht, wie der kleine Ausschnitt rechts neben Figur 5 zeigt, und über ein Glasfaserbündel 58, dass an seinem markierungszugewandten Ende gewissermaßen einen Lesekopf bildet, erkennbar. Dazu kann das Glasfaserbündel 58 über eine nicht dargestellte Lichtquelle, etwa eine LED oder Laserdiode beleuchtet sein. Insbesondere kann der Barcode, statt in einem Zug, quasi parallel, als Bild erfasst und dann elektronisch ausgewertet zu werden, auch quasi seriell durch Abscannen unter Verwendung der Mehrzahl Glasfasern (also der Glasfasern der Reihe nach) ausgelesen werden.

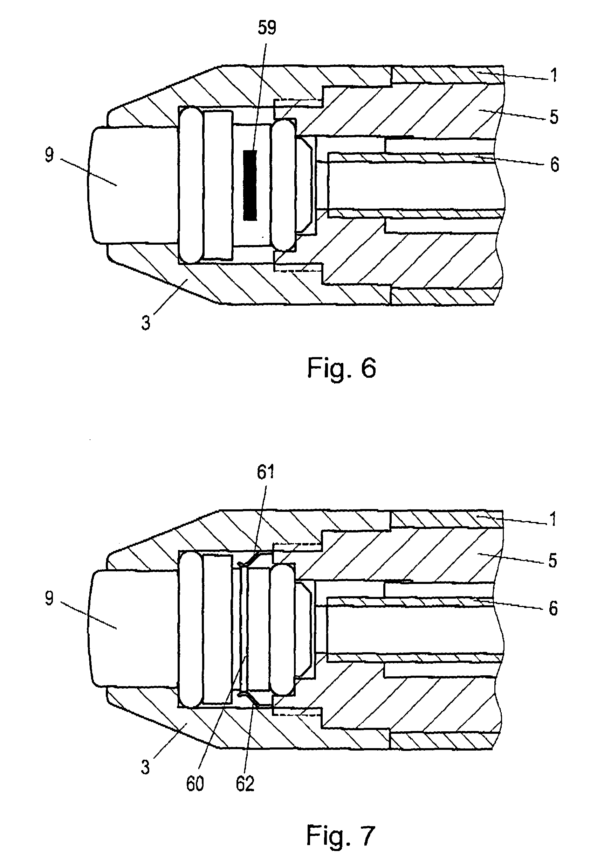

Figur 6 zeigt ein weiteres Ausführungsbeispiel, bei dem der Prallkörper 9 einen auf seiner Mantelfläche angebrachten Barcode 59 aufweist. Dieser Barcode ist in Figur 6 nur symbolisch als schwarzer Streifen 59 dargestellt. Er ist dazu vorgesehen, vor Montage des Prallkörpers 9 durch ein Lesegerät erkannt zu werden, das in dem bereits mehrfach erwähnten Basisgerät eingebaut ist. Hier kommt es also darauf an, dass der Benutzer sicherstellt, tatsächlich den erkannten Prallkörper 9 und keinen anderen zu montieren und, bereits als Voraussetzung hierfür, beim Wechsein eines Prallkörpers 9 tatsächlich auf eine neue Erkennung zu achten.

Das Ausführungsbeispiel in Figur 7 ist nicht auf eine optische Erkennung sondern eine elektrische gerichtet. Hierzu ist ein auf derselben Umfangsfläche des Prallkörpers 9 wie im vorherigen Ausführungsbeispiel umlaufender Widerstandsstreifen 60 vorgesehen, der über Kontaktfedern 61 und 62 kontaktiert werden kann. Durch unterschiedliche elektrische Widerstandswerte des Widerstandsstreifen 60 zwischen den Abgriffsstellen der Federkontakte 61 und 62 lassen sich unterschiedliche Prallkörpertypen erkennen und unterscheiden.

Bei einer nicht dargestellten Ausführungsform könnten in dieser Weise auch Speicher wie beispielsweise ein EEPROM eingesetzt werden, die durch entsprechende Kontakte ausgelesen werden können.

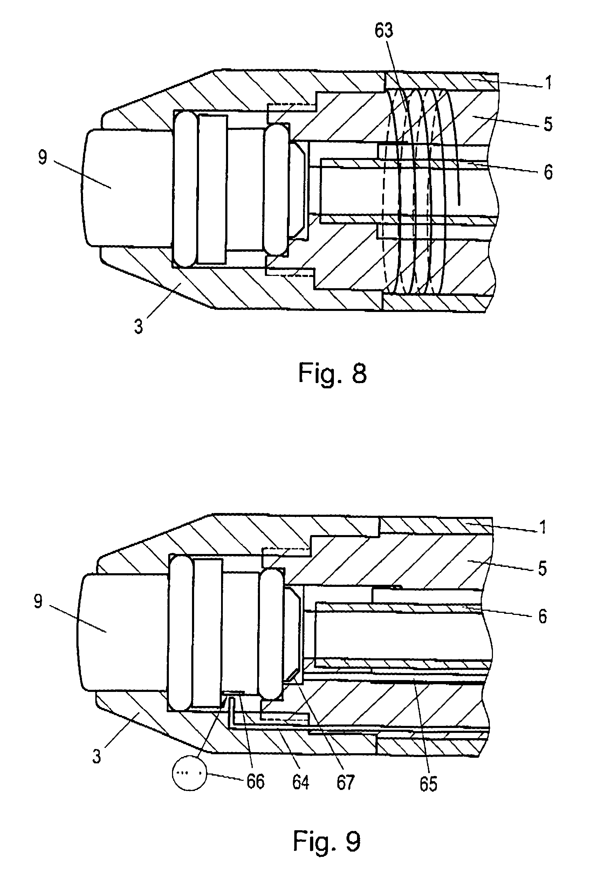

Figur 8 zeigt wieder eine Spule, hier allerdings eine Detektorspule 63. Sie dient zur induktiven Bestimmung des Prallkörpertyps durch Ermittlung seiner magnetischen Impedanz. Dies setzt ausreichend deutliche Unterschiede zwischen den in Frage kommenden Prallkörpertypen hinsichtlich Material und/oder Größe und/oder Form voraus. Für die Montage der Detektorspule 63 gelten die Erläuterungen zur Empfangs/Sendespule 53 in Figur 2.

Das letzte Ausführungsbeispiel in Figur 9 bezieht sich wieder auf eine optische Erkennung wie in Figur 5.

Hier sind zwei Lichtleiter 64 und 65 vorgesehen, ähnlich wie in Figur 5. Beide sind lichtwellenleitende Glasfaserbündel. Der Lichtleiter 64 läuft in seinem letzten Stück durch die Applikatorkappe 3 und weist also eine nicht näher dargestellte optische Koppelstelle zwischen Applikatorkappe 3 und Einsatz 5 auf.

Der zweite Lichtleiter 65 läuft weiter innen und ähnlich wie der Lichtleiter 58 aus Figur 5. Hier werden Punktfolgen als Markierung abgetastet, die beispielhaft bei der Markierung 66 dargestellt sind, und bei der dem anderen Lichtleiter 65 zugeordneten Markierung 67 in entsprechender Weise an der Fase des Prallkörpers 9 vorhanden sind. Dabei bilden die beiden Markierungen und die beiden Lichtielter Alternativen, die aber auch in Kombination vorgesehen sein können, und verdeutlichen die unterschiedlichen geometrischen Möglichkeiten, insbesondere die Anbringung auf einer Mantelfläche wie bei der Markierung 66, die Anbringung auf einer schrägen Fläche wie bei der Markierung 67 (und natürlich die Anbringung an einer Stirnfläche wie 57 in Figur 5). Beispielsweise können die dargestellten vier schwarzen Punkte zusammen mit der Lücke ein Startbit und weitere vier informationsbits darstellen. Auf das Startbit kann auch verzichtet werden, wenn die drehsinngenaue Montage des Prallkörpers 9 ausreichend präzise funktioniert. Dazu können die bereits erläuterten Formschlüsse dienen, auch in Form von Schrägen an dem Prallkörper 9, die nur eine bestimmte drehsinnrichtige Montage ermöglichen, weil sie Vorsprüngen an der Applikatorkappe 3 oder dem Einsatz 5 entsprechen.

Allen bisher gezeigten Ausführungsformen ist gemeinsam, dass die dargestellten Prallkörper 9 und bei bestimmten Ausführungen auch die Applikatorkappen 3 nach der Behandlung eines bestimmten Patienten und vor der Behandlung des nächsten entnommen und gegen einen fabrikneuen Prallkörper 9 ausgetauscht werden. Dabei können behandlungsabhängig verschiedene Prallkörpertypen eingesetzt werden, insbesondere aus verschiedenen Materialien und solchen mit oder ohne fokussierende Wirkung. Insbesondere bei den Ausführungsformen mit geringem technischen Aufwand und Wert der Markierung des Prallkörpers 9 (und solchen ohne Markierung) können gebrauchte Prallkörper 9 weggeworfen werden oder beispielsweise zum Aluminiumrecycling gebracht werden.

## Patentansprüche

1. Verfahren zur regelmäßigen Vorbereitung eines Geräts zur Behandlung des menschlichen oder tierischen Körpers durch mechanische Druckwellen auf eine solche Behandlung, welches Gerät aufweist:
ein bewegbares Schlagteil (13) und
einen Prallkörper (9), der austauschbar ist, wobei die Druckwellen durch Beschleunigen und Aufprallen des Schlagteils (13) auf den Prallkörper (9) erzeugbar und durch extrakorporales Aufsetzen auf die Körperoberfläche einkoppelbar sind,
**dadurch gekennzeichnet, dass** der Prallkörper (9) vor jeder Behandlung eines weiteren Patienten gegen einen unbenutzten Prallkörper (9) ausgetauscht wird.

2. Verfahren nach Anspruch 1, bei dem zumindest einer der Prallkörper (9) aus Kunststoff, insbesondere Polykarbonat oder PEEK, Metall, insbesondere Aluminium oder Edelstahl, oder aus gesinterter Keramik, insbesondere Zirkonoxid, Siliziumoxid oder Siliziumnitrid, besteht.

3. Verfahren nach Anspruch 2, bei dem die Keramik eine Dichte von höchstens 6 g/cm³ aufweist.

4. Verfahren nach Anspruch 2 oder 3, bei dem die Keramik eine Schlagzähigkeit von mindestens 3 MPam und eine Druckfestigkeit von mindestens 2000 MPa aufweist.

5. Verfahren nach Anspruch 2, 3 oder 4, bei dem die Keramik gefärbt ist.

6. Verfahren nach einem der vorstehenden Ansprüche, bei dem das Gerät eine Einrichtung zur automatischen Erkennung von Prallkörpern (9) aufweist, wobei ein Prallkörper erkannt und durch die Erkennung von anderen einsetzbaren, aber typverschiedenen Prallkörpern unterschieden wird.

7. Verfahren nach Anspruch 6, bei dem der Prallkörper (9) eine durch die Erkennungseinrichtung (51 - 62, 64 - 67) erkennbare Markierung (51, 54, 55, 57, 59, 60, 66. 67) aufweist.

8. Verfahren nach Anspruch 7, bei dem die Markierung (60) über einen elektrischen Kontakt (61, 62) erkannt wird.

9. Verfahren nach Anspruch 7, bei dem die Markierung (57, 66, 67) über Licht einschließlich Infrarotstrahlung erkannt wird.

10. Verfahren nach Anspruch 7, bei dem die Markierung (51) über Radiofrequenzwellen ausgelesen wird.

11. Verfahren nach Anspruch 6, bei dem der Prallkörper (9) keine Markierung aufweist und die Erkennungseinrichtung (63) den Prallkörper anhand elektromagnetischer Eigenschaften erkennt; insbesondere anhand einer magnetischen Induktivitätsmessung.

12. Verfahren nach einem der Ansprüche 6 - 11, bei dem hinsichtlich des Typs des erkannten Prallkörpers (9) geeignete Betriebsparameter für die Behandlung kontrolliert oder selbsttätig eingestellt werden.

13. Verfahren nach einem der Ansprüche 6 - 12, bei dem anhand der Erkennung des Prallkörpers (9) das Einsetzen eines unbenutzten Prallkörpers (9) überwacht wird.

14. Verfahren nach einem der vorstehenden Ansprüche, bei dem neben dem Prallkörper (9) vor jeder Behandlung eines weiteren Patienten zusätzlich eine zu seiner Montage dienende Kappe (3) mit ausgetauscht wird.

15. Gerät zur Behandlung des menschlichen oder tierischen Körpers durch mechanische Druckwellen, ausgelegt für ein Verfahren nach einem der Ansprüche 1 - 14, mit:
einem bewegbaren Schlagteil (13) und
einem Prallkörper (9), der austauschbar ist, wobei die Druckwellen durch Beschleunigen und Aufprallen des Schlagteils (13) auf den Prallkörper (9) erzeugbar und durch extrakorporales Aufsetzen auf die Körperoberfläche einkoppelbar sind,
**dadurch gekennzeichnet, dass** der Prallkörper (9) ein für die Behandlung genau eines Patienten ausgelegter Einmalprallkörper (9) Ist.
